# EUROPEAN PATENT APPLICATION

(11) **EP 2 428 564 A1**
(43) Date of publication of application: **14.03.2012**
(21) Application number: 09844226.2
(22) Date of filing: 20.10.2009
(51) Int. Cl.: C12N 7/00, C12Q 1/70, C12Q 1/68, C12N 15/01, C12N 15/51, C12R 1/92

(54) **A HEPATITIS B VIRUS MUTATION STRAIN WITH RESISTANCE TO ADEFOVIR DIPIVOXIL AND THE USES THEREOF**

(30) Priority: 04.05.2009 CN 200910083422
(71) Applicant: Peking University People's Hospital, Beijing 100044 (CN)
(72) Inventor: WEI, Lai, Xicheng District Beijing 100044 (CN); DU, Shaocai, Xicheng District Beijing 100044 (CN); LIU, Lijun, Xicheng District Beijing 100044 (CN); WANG, Jianghua, Xicheng District Beijing 100044 (CN)
(74) Representative: Ricker, Mathias
(86) International application number: PCT/CN2009/001165
(87) International publication number: WO 2010/127474

(57) **Abstract**

A HBV mutation strain is provided, wherein rtE218G mutation occurs at the polymerase region of the mutation strain. The use of the HBV strain in screening anti-HBV drugs and related detection reagents used to detect rtE218G mutation are also provided.

## Description

### Technical Field

This invention relates to the field of pharmaceutical biotechnology, specifically relates to an adefovir-resistant hepatitis B virus (HBV) mutation strain. The invention also relates to the detection reagents thereof and the uses of the virus strain in drug resistance detection, cross-resistance analysis, drug screening and discovery of new drugs and the like.

### Background Art

Adefovir dipivoxil is the prodrug of adefovir (ADV), one of the adenine phosphate compounds, which can be rapidly hydrolyzed into ADV upon oral administration and can play an antiviral role. ADV is a broad-spectrum antiviral drug, which is an inhibitor of nucleoside virus reverse transcriptase and plays an antiviral role by inhibiting HBV replication. Research shows that ADV has a good clinical efficacy and safety on HbeAg-positive or HBeAg-negative patients with chronic hepatitis B. ADV has been used as the first-line drug for chronic hepatitis B antiviral therapy.

Like other nucleoside drugs, drug-resistant mutations of HBV DNA may occur in treatment of ADV and result in drug resistance of ADV. Researches have shown that, when patients with chronic hepatitis B are treated with ADV, the incidence of drug-resistant mutations during 1 to 5 years are 0%, 3%, 11%, 18% and 29%, respectively.

The most common ADV-resistant mutations are rtN236T at D region and rtA181V/T at B region of HBV polymerase. Several mutations may occur together or appear alone at the beginning of treatment with ADV. However, as the increase of the courses of the treatment, rtA181V often occurs accompanying with rtN236T. In the *in vitro* susceptibility tests, compared with wild strains, the sensitivity of rtN236T mutaion strain to ADV is decreased by 3.9 to 13.8 folds, while the sensitivity of rtA181V mutaion strain to ADV is decreased by 2.5 to 3.0 folds. A number of researches have pointed out that, the appearance of rtN236T changed the polarity of amino acids at replication catalytic activity region, resulting in the change of HBV polymerase folding. That is disadvantageous to the binding of HBV polymerase and substrate in the replication phase, and thereby blocks the replication process. The appearance of rtA181V partly corrects the change in the polarity of amino acids, making polymerase folding closer to the initial state after rtN236T mutation, which is conducive to the replication passage of drug-resistant mutation virus strain. In addition, the data of both *in vivo* and *in vitro* experiments suggested that, the sensitivity of rtA181V mutation to lamivudine is less than that of rtN236T mutation. This is probably because rtA181V mutation is adjacent to lamivudine rtL180M, and the occurrence of any mutation will have an ortho-position effect on it.

The effects of rtN236T mutation and rtA181V mutation on HBeAg-negative and HBeAg -positive patients are not exactly the same. The incidence of ADV drug resistance in minor viremia and HBeAg-positive patients is very low, and the resistance is partly reflected in the increase of ALT. For HBeAg-negative patients, if the drug withdrawal occurs after 48 weeks of ADV treatment, the treatment benefit often disappears after 1 to 2 years. However, if drug treatment continues up to 144 weeks, various improvements in virology, serum biochemistry and histology obtained by the patient after 48 weeks treatment will remain and get better. The incidence of drug resistance during this period is very low and the negative effects are similar to that of the initial 48-week treatment.

Currently, ADV is often used in the patients with lamivudine failure during initial treatment. Early period of 48 weeks and 96 weeks clinical trials suggested that both wild-type and YMDD mutant strains are sensitive to ADV, and HBV DNA decreases exponentially after treatment, as well as being well tolerated by patients. Therefore, the combination of two drugs is more effective in inhibiting viral replication, but the combination of mutations of rtL180M + M204V and rtN236T may also occur at polymerase region, resulting in double resistance to lamivudine and ADV. Although studies have shown that lamivudine-resistant strain is sensitive to ADV, for HBV itself, the combination of the three mutations increase the level of ADV-resistance, and the sensitivity to ADV has decreased by 2 folds compared with rtN236T single mutation. The combination of mutations in molecular level results in more difficulty of the combination of substrate binding sites and adefovir, thus further decreasing the sensitivity to ADV.

Up to now, researchers have found that not only rtA181V and rtN236T mutations at HBV polymerase gene region may lead to ADV-related resistance, other mutations associated with ADV, such as rtI233V, rtS85A, rtS85P, rtS119A, rtH133L, rtV214A, rtH234Q, rtP237H and rtN238D etc., may also be related to ADV resistance. However, ADV resistance in clinical therapy still appears in many patients with chronic hepatitis B, and the existance of known mutation sites can not be detected. Therefore, ADV-related HBV mutations is still required to be further explored.

### Summary of the Invention

The object of the present invention is to find new HBV mutation sites causing ADV resistance and the corresponding HBV mutation strains, provide the detection methods of the new mutation sites and the test kits thereof, provide the methods for identification of the HBV mutation strains and identification kits thereof, and provide the uses of the new mutation sites and the corresponding HBV mutation strains in screening anti-HBV drugs, monitoring drug resistant mutations and the like.

The inventor of the present invention found that rtE218G mutation, i.e. the amino acid codon at position 218 of HBV DNA polymerase region is mutated from GAG to GGG, and this mutation is a new HBV gene mutation site associated with ADV resistance. The results of the phenotype experiment *in vitro* showed that rtE218G mutant virus strain can cause ADV-resistance independently, and the IC₅₀ of rtE218G mutation is 5.5 folds of that of the wild virus strain. The replication capacity of rtE218G mutation strain *in vitro* is reduced to about 87% of that of the wild strains.

The invention also provides the uses of rtE218G mutation strain in screening antiviral drugs. The mutation strain can be used for cross-resistance analysis of other anti-HBV drugs and drugs screening. The skilled person in the art may further clone the DNA fragments containing rtE218G mutation site into a vector, and may further introduce the vector into corresponding host cells for the convenience of use and storage.

The present invention also provides several methods for detecting the rtE218G mutation site and identifying the corresponding HBV mutation strains, which includes the following:
1. Specific probe hybridization method: the probes directed to rtE218G mutation site are used to hybrid with the HBV DNA fragments to be detected, determining whether the rtE218G mutation occurred by detecting the results of hybridization. Reverse probe hybridization can also be used, including the steps of: fixing the probe on a solid phase vector, hybridizing the labeled DNA fragments to be detected with the probe, washing the unhybridized fragments, and detecting the results of hybridization. According to this principle, the following two methods can be further used for detecting hybridization:
   1) Reverse linear probe hybridization method: Based on the principle of probe hybridization, firstly a series of probes covering the region of high incidence of mutations are designed and synthesized, wherein S series probes are directed to the wild-type sequences and R series probes contain a number of probes with common mutation sequences, and a genus-specific probe can also be designed. The series of probes in turn are fixed on a hybrid membrane, and hybridized with affinity-labeled PCR product under strict conditions, followed by detecting hybridization signals, and determining whether a mutation occurs and its general location according to the different hybrid band spectrum. Based on the principle of reverse hybridization, the amplified HBV DNA product with biotin-labeled was hybridized with specific oligonucleotide probe fixed on nylon membrane strips in the form of parallel lines. After hybridization, unhybridized DNAs are eluted from the detection strips. Subsequently, streptavidin labeled with alkaline phosphatase are added thereto, and bond with biotin labeled on the hybridization product. Finally, BCIP/NBT is added to develop color (appearing purple/brown) for determining the results.
   2) Gene chips method: According to the hybrid principle, i.e., the principle of complementary DNA base pairing, double-stranded DNA molecules were formed at room temperature and neutral conditions. However, under high temperature condition, or in the presence of organic solvents or alkali, the hydrogen bonds between double helix break, and the double helix unlocks to form single-stranded molecules (it is called DNA denaturation, and the temperature of DNA denaturation is called Tm value). The denatured DNA is of decreased viscosity, increased sedimentation velocity, increased buoyancy and increased UV absorption. When the denaturing conditions eliminated, two complementary strands of the denatured DNA may re-bind and restore the original double helix, this process is called renaturation. The physical and chemical properties of the renatured DNA can be restored. By making use of the important physical and chemical properties of DNA, heterologous hybrid molecules will be formed during the renaturation between two or more polynucleotide chains with complementarity which come from different sources, this process is called hybridization. The molecules of a hybrid do not come from the same dimer. Since temperature is easier to be controlled than the other denaturation means, double-stranded nucleic acids will unwind to single-stranded molecules at the temperature above its denaturation temperature (Tm value), while the single-stranded molecules will renature to double-stranded molecules according to the principle of base pairing at the temperature below the Tm value. Thus, the nucleic acids hybridization was often carried out in the processes of DNA denaturation and renaturation caused by temperature changes.

   If there exist the complementary base sequences between the single-stranded nucleic acid molecules, a stable double-stranded region can appear by forming non-covalent bonds between base pairs, that is the basis of nucleic acid hybridization. Since the sequences of two single-stranded nucleotides are not necessary to be completely complementary to each other to form a hybrid molecule, if there is a certain extent of complementary sequences between the single-stranded nucleic acids from different sources, hybrid double strands can be formed, and molecular hybridization may occur between two single strands of DNA and DNA, RNA and RNA or RNA and DNA. By making use of this feature of molecular hybridization, firstly, one of the hybrid chains is labeled with a marker which could be detected by a certain means, and hybridized with another nucleic acid (sample to be detected), qualitative or quantitative detection of the nucleic acid sequences is then carried out to analyze if the gene exists in the sample to be detected or whether the gene expression changes. Reverse hybridization method is often used for gene chips, that is, a plurality of probe molecules are arrayed on the chip, and the labeled target nucleic acid samples are hybridized with the chip. The advantage is that thousands of targets even the whole genome as the target sequences may be researched simultaneously.
2. Polymerase chain reaction-restriction fragment length polymorphism (PCR-RFLP) analysis: the principle is: firstly amplifying the target DNA. fragments by PCR (polymerase chain reaction), then digesting the DNA fragments to be detected with restriction endonuclease, wherein the restriction endonuclease recognizes and cuts the specific sequence, and then subjecting the digestion products to electrophoresis, followed by analyzing the specific endonuclease sites of the sequence of this section by the restriction enzyme map, comparing the differences of gene sequences from different sources by the diversity of the fragments. Herein, rtE218G mutation nucleotides are included in the recognition sequence of restriction endonuclease. In one embodiment according to the present invention, sequences of reference strains of HBV genotypes are selected from the GenBank, mutagenic primer B2 (downstream 5'-TTG GTA ATA GAG GTA AAA AGG TAC-3') is designed, the primer together with the rtE218G mutation site constitute the endonuclease cutting site which can be identified by *Ban* I restriction endonuclease. HBV DNA is amplified by using B1 (upstream 5'-GAG TGG GCC TCA GTC CGT TTC TC-3') and B2 to obtain 160bp amplified fragments, then digesting them with *Ban* I restriction endonuclease, the 160bp amplified fragments of rtE218G mutation is cut into two fragments of 140bp and 20bp, while that of the wild-type strain can not be cut by *Ban* I and remains as 160bp fragments.
3. Ligase chain reaction (LCR), which is also a technology of DNA amplification and detection *in vitro,* is mainly used for detecting point mutations. The basic principle of LCR is: rapidly amplifying DNA fragments by connecting with two adjacent oligonucleotides chains complementary to the template DNA in the presence of DNA ligase. The two adjacent oligonucleotide fragments complementary to the template DNA strands can be linked by DNA ligase. The base mismatch at the joint of the two oligonucleotides may prevent the reaction. So it can clearly distinguish if the oligonucleotides are completely complementary to the template DNA strands to detect the point mutation of gene by LCR. The program is as follows: under appropriate reaction conditions, firstly, template DNA, DNA ligase, and oligonucleotide primers are heated to a certain temperature (94-95°C) to denature DNA and open the double-strands, and then annealed (about 65°C); the primers are bound to the complementary template DNA and a gap is left, if the adjacent oligonucleotide primers which are hybridized with the target sequences are completely complementary to the target sequences, DNA ligase can ligate them and close the gap, then three steps (denaturation - annealing - ligation) of the LCR reaction can be carried out repeatedly, with the products of each ligation reaction as the templates of the next cycle, so that more oligonucleotides could be ligated and amplified. If there is a point mutation at the joint of the target sequence, primers can not be bond precisely with the target sequences, the spatial structure of the nucleotides near the gap changes, and the ligation response can not be carried out, thus forming no ligated product.
   The primers in LCR are two pairs of complementary primers, with the primer length of 20bp to 26bp, which could ensure the specific binding of the primer and target sequence. The specificity of identifying point mutations by LCR is higher than that of identifying point mutations by PCR, the specificity firstly depends on the specific binding of primer and template, then on the specificity of the heat-resistant ligase. The temperature of LCR ligation reaction is close to the melting temperature of oligonucleotide (Tm), therefore the specificity of identifying the single nucleotide mismatch by LCR is extremely high.
   The amplification efficiency of LCR is equal to that of PCR, and there is only two temperature cycles needed by using the heat-resistant ligase to carry out LCR, i.e. denaturing at 94°C and renaturing and ligating at 65°C, with about 30 cycles, and the detection of its products are more convenient and sensitive. For now, the method is mainly used in the research and detection of point mutations, detection and directed mutagenesis of microbial pathogens, etc., and it can also be used in the diagnosis of polymorphism and the products of single base genetic diseases, the species identification of microbiology, and the research of point mutation of cancer gene.
   According to the method of LCR, two adjacent oligonucleotide chains complementary to the DNA sequences of HBV mutation strain are designed for rtE218G mutation, one oligonucleotide chain of each pairs is adjacent to another chain, the terminal bases of the adjacent ends are complementary to the bases of the rtE218G mutation site. Ligase chain reaction is carried out by taking the HBV DNA to be detected as templates, followed by determining whether rtE218G mutation occurred by detecting the ligation products.
4. Nucleotide sequencing method: The target DNA fragments are amplified by PCR to obtain the DNA fragments to be detected, sequencing nucleotide directly to determine whether the mutation occurred. PCR products can be cloned firstly, and then several colonies are picked and sequenced.
5. Sequence-specific primer method: Sequence-specific primers are designed and synthesized according to the mutation sites. The target DNA fragments are amplified by PCR, determining whether it was the mutation strain by detecting the presence or absence of specific PCR products since the wild or the mutant sequence only complementarily binding to the corresponding primers to amplify. Preferably, probes are bond to carry out quantitative PCR. In one embodiment according to the present invention, specific primers F1: 5'-GAG TGG GCC TCA GTC CGT TTC TC-3', F2: 5'-TTG GTA ATA GAG GTA AAA AGG TTT C-3', and fluorescent labeled probe: FAM-5'-TAG TGC CAT TTG TTC AGT GGT TCG TAG-3'-TAMRA are used to carry out quantitative PCR detection, specific amplification can be detected in only mutation strains.
6. Single Strand Conformation Polymorphism Analysis of Polymerase Chain Reaction Products (PCR-SSCP) is a recently developed genetic analysis method.

The basic procedures of PCR-SSCP analysis are as follows: Firstly, specific target sequences are amplified by PCR, followed by denaturing the amplified products to single-strands to conduct non-denaturing polyacrylamide gel electrophoresis. When electrophoresis is carried out in the neutral polyacrylamide gel without denaturant, the migration rate of DNA single strand not only is related to the length of DNA chain, but also depends mainly on the conformation of DNA single-strand. Under non-denaturing conditions, DNA single strand itself can fold into a conformation with certain spatial structure. This conformation depends on the bases of DNA single strand, the stability of the conformation is maintained by the interactions (mainly hydrogen bonds) between intramolecular partial sequences. The DNA single strands with the same length and different sequences or even a different single base may form different conformation, and have different electrophoretic migration rates. After the PCR products are denatured, the single-stranded products are subjected to neutral polyacrylamide gel electrophoresis; when changes such as base substitution, or insertion or deletion of several bases in the target DNA happened, mobility shift may occur due to the change of migration rate, which can distinguish mutant DNA from normal DNA. Thus, PCR-SSCP analysis is a technology of DNA single strand gel electrophoresis, which can detect gene variation based on the changes of electrophoresis migration rate of equal length single-stranded DNA with different conformations in a neutral polyacrylamide gel electrophoresis. The technology has been widely used in detecting mutation of cancer gene and anti-cancer gene, disease-causing gene analysis, gene diagnosis of genetic disease, gene mapping and other fields.

In order to show the results of SSCP analysis with high specificity and sensitivity, a variety of PCR-SSCP techniques have been developed, and each has its own advantages and fields of application. For example, isotopes or fluorescein etc. can be used to label primers or nucleotides in PCR amplification, and silver staining or ethidium bromide staining can also be used to show results after electrophoresis. The most commonly used radioisotope PCR-SSCP method is to be emphatically introduced herein. During amplifying specific target genes sequence by PCR, γ-32P-ATP-labeled primers are used or α-32P-dCTP are added directly in the PCR reaction system for PCR amplification, so that the amplified products are labeled with isotope markers, and then the amplified products are denatured to single strand to conduct non-denaturing polyacrylamide gel electrophoresis, and the results are displayed by autoradiography. The PCR amplification products are labeled with isotope markers by primers labeling or bases incorporation method, the product signals can be enhanced for several orders of magnitude. Comparing the two methods, the former is economic and of high amplification specificity, which is often used for detecting and screening the large samples; while the latter is simple and suitable to carry out in a general laboratory, which can be used for detecting and screening small samples or large samples.

The invention further provides detection reagents for detecting rtE218G mutation sites and identifying the corresponding HBV mutation strains, comprising a variety of primers and/or probes. The detection reagents can be further used by assembling with other necessary reagents into a test kit for easy use. For example, the above primers and restriction endonuclease are used to constitute a PCR-RFLP test kit, or site-specific primers and fluorescent probes are used to constitute a fluorescent quantitative PCR test kit, and so on.

Specifically, the detection reagents are selected from the group consisting of:
1) primer pairs, either (a) which specifically amplifies nucleotide sequences containing rtE218G mutation sites; or (b) whose amplified products contain rtE218G mutation sites which may constitute the identification sites of the restriction endonuclease with the sequence of one end or the sequences of both ends;
2) sequence-specific primer pairs, one of which specifically binds to the nucleotide sequence containing rtE218G mutation;
3) specific probes, which specifically bind to the nucleotide sequence containing rtE218G mutation;
4) oligonucleotide sequences for ligase chain reaction, wherein as per the practice of LCR, two pairs of adjacent oligonucleotide chains complementary to the DNA sequences of HBV mutation strain respectively are designed for rtE218G mutation, one oligonucleotide chain of each pair is adjacent to another, and the terminal bases of the adjacent ends are complementary to the bases of rtE218G mutation sites.

The kit further comprises the restriction endonuclease when it contains the primer pairs in 1); or the kit further comprises the probes specific binding to the target sequences when it contains the sequence-specific primer pairs in 2).

The methods for the detection of HBV rtE218G mutations and identification of corresponding HBV mutation strains can be used only for laboratory research, or only for detection or identification of HBV rtE218G mutation sites and the corresponding HBV mutation strains for monitoring drug resistance and developing of new drugs. HBV rtE218G mutation strains can also be used to guide clinical administration. The present invention also provides a method for clinical administration guidance, including detection of HBV rtE218G mutation, and choosing of drugs based on detection results. Of course, it is preferred to use the above reagents or kits for detecting HBV rtE218G mutations. According to the present invention, the surprisingly discovery of rtE218G mutation sites can be bond with known variable sites to explain the clinical phenomenon of resistance to ADV. The detection of the mutation sites can be used in guiding clinical administration, developing and screening of new drugs.

### Description of Drawings

Figure 1 shows the 1.2 folds HBV full-length gene (about 3.8kb) and the PUC18 vector fragment (about 2.8kb) generated after double digestion of plasmid with endonuclease *EcoR* I and *Hin*d III;
Figure 2 shows the mutant plasmid sequencing map which verifies the correct introduction of the mutation part, wherein the position of the mutagenic nucleotide is indicated by an arrow and the underlined is the codon encoding corresponding mutagenic amino acid;
Figure 3 shows the result of the Southern blotting, which indicates the inhibition effects of ADV with different concentrations on the replication of the intermediates of wild strain and rtE218G mutant strain of HBV;
Figure 4 shows dose-response curve obtained by the results of Southern blotting based on gray value analysis, which indicates the different inhibition effects of ADV on wild strain and rtE218G mutation strain, the IC₅₀ of mutation strain is 5.5 times that of the wild strain.

### Mode for Carrying Out the Embodiment

Examples are described below to further illustrate the contents of the invention, but they should not be construed as limiting the invention. All the modifications and variations of the methods, steps or conditions of the invention without departing from the spirit and nature thereof may belong to the scope of the invention.

Unless otherwise indicated, the technique means employed by the invention are conventional techniques well known to the skilled in the art.

### Example 1

### I. Experimental materials

### 1. Serum

Serum samples from the ineffective ADV-therapy patients with chronic hepatitis B were stored at -30°C for later use.

### 2. Plasmids and recipient strains

Plasmid PUC-HBV 1.2WT comprising 1.2 copies of HBV DNA (genotype C, Genbank Accession No. AY518556) was deposited at Institute of Liver Diseases, PEKING UNIVERSITY PEOPLE'S HOSPITAL. pGEM-T Easy Vector Kit for A-T cloning was purchased from Promega Corporation(US), and DH5α competent cells were purchased from DingGuo Biotech.Co.,Ltd (China).

### 3. Major reagents

Proteinase K, poly(A), dNTPs and glycogen were purchased from Boehringer Mannheim Corporation ( BM ) (DE); X-gal and IPTG were purchased from Promega Corporation(US); agarose and diethylprocarbonate ( DEPC ) were purchased from Fluca Corporation; tryptone and yeast extract were purchased from Oxid Corporation; phenol was purchased from Invitrogen Corporation; DMEM medium and fetal bovine serum were purchased from GIBCO Company; nitrophenol phosphate(pNPP) was purchased from Sigma Corporation, and other biochemical reagents were analytical reagents made in China.

QIA quick Gel Extraction Kit and EndoFree TM Plasmid Mega Kit were products of Qiagen Corporation (DE); efficient eukaryotic transfection reagents VigoFect was purchased from Vigorous Biotechnology (Beijing) Co., Ltd.; HBsAg and HBeAg were determined by ARCHITECT ® Microparticle Chemiluminescence Detection Kit (Abbott Corporation, US); HBV DNA RT-PCR Kit was purchased from Piji Biological Engineering Co., Ltd., Shenzhen; Digoxigenin Probe Labeling Kit, anti-digoxin antibody and CPD Star Kit were purchased from Roche Corporation (DE).

### 4. Tool enzyme

High-fidelity DNA polymerase was purchased from Quanshijin Biological Technology Co., Ltd., Beijing; T4 DNA ligase was purchased from Promega Corporation (US); all kinds of restriction endonucleases were purchased from NEB Corporation (US).

### 5. Hepatoma cell strain

Human hepatoma cell line HepG2 were purchased from Institute of Biochemistry and Cell Biology, Chinese Academy of Sciences, and were frozen in liquid nitrogen. After recovery, the cells were incubated in an incubator at 37°C, in 5% CO₂ in high glucose DMEM medium containing 10% fetal bovine serum.

### 6. Adefovir dipivoxil

Adefovir dipivoxil, the lyophilized powder was stored at 4°C. After being dissolved in PBS, it was stored at -20°C.

### 7. Major equipments and supplies

Low-temperature high-speed centrifuge CF15R was the product of HITACHI Corporation; high-speed desk centrifuge Micromax was the product of IEC Corporation ( DE ); PTC-100 Peltier Thermal Cycler PCR instrument was the product of MJ Research Corporation( US ); automatic gel imaging system GEL Doc 2000 was the product of Bio-Rad Corporation; constant temperature incubator was the product of Revco Corporation ( US ) ; constant temperature air bath shaker was the product of Dongming Medical Instrument Factory, Haerbin (China); carbon dioxide incubator 3111 was the product of Forma Scientific company; clean benches were the products of Great Wall Air Purification Engineering company, Changping, Beijing; autoclave steamer MLS-3000 was the product of SANYO Corporation; enzyme-labeling instrument Reader 230s was the product of DRGA non Teknika Corporation; RT-PCR instrument LightCycler II was the product of Roche Corporation.

### 8. Designing primer and sequencing

Primers employed in the detection method according to the invention were designed on the basis of the sequence of each HBV genotype in the GenBank, and mutation sites were introduced into the corresponding positions; the rest primers were designed on the basis of the sequence of HBV in PUC-HBV1.2WT. All the primers were synthesized by Sanboyuanzhi Biotechnology Company, Beijing. Sequencing was performed by Sino Genome Research Center Co., Ltd., Beijing. Reagents used in sequencing was ABI BigDye3.1, sequencing instrument was ABI 3730XL.

### II. Experimental procedures

### 1. Serum samples of the ADV-resistance patients with chronic hepatitis B were

### amplified at the polymerase region, and the PCR product was cloned and sequenced.

The virus DNA from the serum samples was extracted by proteinase K-phenol-chloroform method, and then the DNA was dissolved in 30µl TE and stored at -80°C for later use.

Each PCR reaction system had a total volume of 30µl and consisted of 3µl of 10×PCR buffer, 1µl of 10mmol/L dNTP, 50ng of the upstream primer and the downstream primer respectively, 1µ of high-fidelity Taq polymerases, finally, according to the amount of the templates, DEPC water was added to make a total volume of 30µl.

First, 3µl of HBV DNA extracted from the serum of individual patient was taken as template, and BSE (upstream: 5'-CTC GTG GTG GAC TTC TCT CA-3 ') and P0 (downstream: 5'-GGG TTG CGT CAG CAA ACA CTT G-3 ') as primers to conduct amplification, reaction conditions: 94°C, 300s; 94°C, 30s; 55°C, 30s; 72°C, 45s; 30 cycles. 3µl of the first PCR product was taken as template, and Hinfed (upstream: 5'-GAG TGG GCC TCA GTC CGT TTC TC-3') and SSG (downstream: 5'-ACA TAT CCC ATG AAG TTA AG-3') as primers to conduct the second PCR amplification, reaction conditions: 94°C, 300s; 94°C, 30s; 55°C, 30s; 72°C, 45s; 35 cycles. 244bp of the PCR products were obtained. The PCR products without further purification were directly ligated with the pGEM-T Esay Vector at 4°C overnight. Ligation system consists of:

| | |
|---|---|
| 2×Rapid Ligation Buffer | 5.0µl |
| pGEM-T Easy vector ( 50ng/µl ) | 0.5µl |
| T4 DNA ligases ( 3 Weiss unit/µl ) | 0.5µl |
| PCR products | 2-4µl |
| dH₂O supplemented to make up the total reaction volume to | 10.0µl |

100µl of competent *E.coli* DH5α was added to 5µl of the ligation mixture. The mixture was shook gently and then kept on ice for 20min. The mixture was heat shocked for 60s in circulating water bath at 42°C and kept on ice for 2min. 1ml of the LB liquid medium pre-heated at 37°C was added, and then the mixture was incubated with shaking at 37°C, 250rpm for 1h.

Appropriate volume of the transformed products was coated on LB plates(containing 1mol/l IPTG, 4µl; 20mg/ml X-gal, 40µl) with 100mg/ml ampicillin, and incubated upside-down at 37°C overnight.

24 white colonies were picked and inoculated in 2ml of LB liquid medium with 100mg/ml ampicillin, and incubated with shaking at 37°C, 250rpm overnight. 1.5ml of overnight products were transferred to a centrifuge tube and centrifuged at 5,000rpm for 30s, and the supernatant was removed; then the *E.coli* was suspended in 100µl of solution I (50mmol/L glucose, 50mmol/L Tris-HCl (pH8.0), 10mmol/L EDTA), and kept for 5min at room temperature. 200µl of solution II (0.2mol/L NaOH, 1% (WN) SDS) was added thereto, gently mixed upside down for 5-6 times, and kept on ice for 5min. 150µl of solution III (3mol/L KAc, 2mol/L HAc) was added thereto and gently mixed up with shaking, kept on ice for 5min and then centrifuged at 12,000rpm for 5 min. The supernatant was transferred to a new centrifuge tube and 900µl of anhydrous ethanol was added thereto and mixed, followed by standing for 5min at room temperature and then centrifuging at 12,000rpm for 5 min. The supernatant was removed and the precipitates were dried at room temperature. 100µl of solution IV (20µg/ml RNase TE (pH8.0)) was added to suspend the precipitates and form a plasmid solution. *EcoR* I restriction endonuclease was used for digesting and identifying:
The reaction system consists of:

| | |
|---|---|
| 10×Enzyme Buffer | 1.0µl |
| EcoR I ( 10 unit/µl ) | 0.2µl |
| plasmids | 0.2µg |
| dH₂O supplemented to make up the total reaction volume to | 10µl |

The digestion was conducted for Ih at 37°C. Electrophoresis was performed on 1.0% agarose gel, followed by staining with EB and observing the results under UV lamp. According to the length of the inserting fragments, whether the HBV gene insertion exists or not can be determined.

Positive colonies were picked and propagated for extracting the plasmids, followed by performing the forgoing restriction endonuclease analysis and identification, as well as sequencing after analysis and identification.

### 2. Constructing mutagenic plasmids by PCR-mediated site-directed mutagenesis technology

Plasmid PUC-HBV 1.2WT harbors 1.2 copies of HBV DNA (genotype C, Genbank Accession No. AY518556). The first PCR amplification was conducted with 2ng of PUC-HBV1.2WT as template, and two pairs of primers P1A-2 (upstream: 5'-GAACATCGCATCAGGACTCCTAGGACCCCTG-3') and E218-3 (downstream: 5'-GGGTTTAAATGTATACCCAAAGACAAAAGAAAATTGGTAACAGCGGCAT AAAGGGACCCAAG-3'), E218-P1 (upstream: 5'-CAACATCTTGGGTCCCTTTATGC-3') and P2A (downstream: 5'-GGCATTAAAGCAGGATATCCAC-3') were used respectively. The amplification system was the same as that described above, reaction conditions: 94°C, 300s; 94°C, 45s; 55°C, 45s; 72°C, 60s; after 35 cycles, then extension at 72°C for 7 min. 680bp of fragment A and 287bp of fragment B were obtained respectively. Fragments A and B were purified by Gel Purification Kit (Qiagen, Germany), respectively. The purified fragment A and B were mixed together in a ratio of 1:1 and the mixture was diluted in a ratio of 1:100. The second fusion PCR reaction was conducted with 3µl of the mixture as template and primers P1A-2 and P2A; the reaction system was the same as that described above. PCR conditions: 94°C, 300 s; 94°C; 60 s; 55°C, 60s; 72°C, 60 s; after 35 cycles, extension at 72°C for 7 min. 899bp of fragment C was obtained. With A-T cloning (as described above), *EcoR* I digesting and sequencing, successfully mutagenic positive clones were identified. Two restriction endonucleases *EcoR* V and *Avr* II were used to digest the positive clones and the plasmid pUC-HBV 1.2WT, the product was ligated directly and cloned to obtain the mutagenic rtE218G plasmid PUC-HBV1.2-E218G (SEQ ID NO.20). The *E.coli* containing the mutagenic plasmid was kept in China General Microbiological Culture Collection Center (CGMCC) (Address: NO.3 Datun Road, Chaoyang District, Beijing, Institute of Microbiology, Chinese Academy of Sciences, zip code:100101) on May 26, 2009 with the taxonomic name *Escherichia coli* (*E.coli*) PUC-HBV1.2-E218G and the deposit number CGMCC No.3079.

### 3 Test on drug sensitivity

After recovery, Human hepatoma cell strain HepG2 grew as the adherent monolayer cells in the DMEM medium with 10% fetal bovine serum at 37°Cin 5% CO₂. Every 3 to 5 days, cells were passaged one generation in a ratio of 1:3. 24 hours before transfection, cells were inoculated in an appropriate amount (about 2 × 10⁵ per well in a 6-well plate). Preferably, cell density was 40 to 60% for transfection (also, 80 to 90% as well). 1 hour before transfection, the medium was replaced with fresh complete culture medium (2ml per well), and incubated at 37°Cin 5% CO₂. To each well, for example, saline for injection was added to 2µg of DNA to make a total volume of 100 µl, followed by mixing gently and standing at room temperature. Saline for injection was added to 2µl of VigoFect to make a total volume of 100 µl, followed by mixing gently and standing at room temperature for 5 minutes. The diluted VigoFect was added to the diluted DNA solution dropwise, mixed gently, followed by keeping the resultant working solution for transfection stand at room temperature for 15 minutes. The transfection working solution was mixed gently and 2 ml of medium was added thereto dropwise, mixed gently and then incubated at 37°C in 5% CO₂. 2 days after transfection, the medium was replaced and ADV were added thereto with the final concentrations of 0, 0.01, 0.1, 1, 5, 10 and 20µM. Hereafter, the medium was replaced every 2 days and anti-HBV drugs/ADV was added thereto as well. The cells were harvested 8 days after transfection.

### 5. Quantitative detection of HBV DNA in cell culture supernatant

Cell culture supernatant was treated with 100µg/ml DNase I at 37°C for 1 hour to digest the residual transfected plasmid. Virus DNA was detected on Light Cycler II automatic quantitative PCR instrument (Roche, US), using HBVDNA quantitative PCR detection kit. The samples were treated and amplified in accordance with the manufacturer's instructions.

HBsAg and HBeAg were detected using ARCHITECT ® microparticle chemiluminescence detection kit (Abbott, US) and the operating procedures and the calculating methods were employed in accordance with the manufacturer's instructions. HBsAg was represented as IU/ml, and HbeAg was represented as S/CO value.

### 6. Detection of intracellular HBV replication intermediates by Southern blotting

### 6.1 Preparation of whole-genome HBV DNA probe

The preparation of digoxigenin-labeled whole-genome HBV DNA probe by PCR was conducted in accordance with the manufacturer's instructions. The reaction primers: upstream D1, 5'-CCGGAAAGCTTGAGCTCTTCTTTTTCACCTCTGCCTAATCA-3'; downstream D2, 5'-CCGGAAAGCTTGAGCTCTTCAAAAAGTTGCATGGTGCTGG-3'. The reaction conditions: 94°C, predenaturation 2 min; 94°C, 30s; 55°C, 30s; 72°C, 2 min; 35 cycles in total; and from the beginning of the 11th cycle, the extension time was additionally prolonged 20 seconds, and finally extension at 72 °C for 7 minutes. 1µl of 6 x loading buffer and 4µl of H₂O were added to 1µl of the PCR product. Electrophoresis was conducted on 1.0% agarosel, stained with 0.5µg/ml EB and observed under UV lamp. It should be noted that, for the length of the expected product the electrophoresis result showed that the fragment was slightly larger than 3.2kb, since the incorporation of digoxigenin-labeled dUTP fragment might slow down the speed of electrophoresis. The purified PCR product was purified with Qiagen gel purification kit and compared with standard content of probe for quantification.

### 6.2 Extraction of HBV replication intermediates in cytoplasm

The harvested cells were washed twice with PBS, and then 300µl of cell lysis solution was added to each well (50mmol/L Tris-HCl, pH7.4, 1mmol/l EDTA, 1% NP-40) to dissolve and break the cells completely. The lysate was transferred to 1.5ml EP tubes, and kept on ice after shaking for 15 minutes. The EP tubes were shook again and centrifuged at 14,000 rpm for 1 minute to remove the nucleus. The supernatant was transferred to a new centrifuge tube, and then final concentration of 10mol/l MgCl₂, 100µg/ml DNase I were added, and digested at 37°C for 1h to free HBV DNA. Final concentration of 25mmol/L EDTA was added to terminate the DNase I digestion reaction. Final concentration of 0.5mg/ml proteinase K and 1% SDS were added to lyse the cells at 60°C for 1h. The lysate was extracted twice by phenol: chloroform: isoamyl alcohol (25:24:1, V/V). The supernatant was collected, 2.5× volumes of ethanol, 0.25× volumes of 10mol/L ammonium acetate, 1µl of (20mg/ml) glycogen were added thereto, and precipitated at -20°C overnight. The resultant was centrifuged at 4°C, 14000rpm for 15 minutes and the supernatant was removed. The precipitate was washed twice with70% ethanol pre-cooled at 4°C, and centrifuged at 4°C, 14000rpm for 5 minutes. The obtained HBV DNA was dissolved in 10µl of TE containing 20µg/ml of RNase A.

### 6.3 Detection of HBV replication intermediates in cytoplasm by Southern blotting hybridization

Electrophoresis of the obtained HBV replication intermediates in cytoplasm was performed on 1.0% agarose gel. After electrophoresis, the gel was placed in denaturing solution (0.5mol/L NaOH, 1.5mol/L NaCl) with 5 times the volume of the gel, followed by gently shaking on a bleaching shaker for 45min to denature at room temperature. Then the gel was washed with distilled water and transferred into approximately 5× volumes of neutralized solution (1mol/L Tris-HCl, pH7.4, 1.5mol/L NaCl), and neutralized twice for 30min each time. A piece of nylon membrane, 6 pieces of 3MM filter paper which were the same size as the gel were prepared. The nylon membrane and 3MM filter paper were immersed in transferring buffer 0.5×TBE for 5min. Judging from the moving direction of DNA, sponge, three layers of 3MM filter paper, agarose gel, nylon film, three layers of 3MM filter paper, and sponge were laid in turn from negative polar to positive polar with a flat tweezer in an electrotransfer unit. After being aligned, electrophoresis was performed for transferring film under 40 volts of constant voltage at room temperature (<400mA) for 2-4 hours.

Then the electrophoresis was ceased, followed by marking the locations of the wells of loading samples on the gel with a pencil. The nylon membrane was washed in 5× SSC to remove agar debris. Then the nylon membrane was taken out, after absorbing the fluids on the surface of the membrane it was laid on paper tissues and dried at room temperature for 30min. The side of the nylon membrane carrying DNA was exposed to UV under 100mJ/cm² for fixation of UV cross-linking. Subsequently, the fixed nylon membrane was suspended on the surface of 5× SSC, followed by infiltrating completely from bottom to top for 2 min. The infiltrated membrane was transferred into a hybridization flask. Pre-hybridization solution DIG Easy Hyb preheated to 42°C was added thereto in proportion of 100µl (10ml/100cm²) of DIG Easy Hyb for per square centimeter of membrane. Prehybridization was conducted in hybridization chamber at 42°C for 30min.

After pouring off the pre-hybridization solution, hybridization solution Dig High Hyb (3.5ml/100cm²) preheated to 42°C was added to the hybridization flask, then final concentration of 25ng/ml of digoxigenin-labeled HBV genome probe which was denatured at 100°C for 5min was added thereto. The hybridization was conducted in hybridization chamber at 42 °C overnight.

The hybridized membrane was removed to a dish of 15cm diameter, and about 200ml of 2× SSC and 0.1% SDS solution were added. The dish was placed on a bleaching shaker, and shook gently at room temperature for 5min × 2.

After pouring off the eluant, 200ml of 0.5× SSC and 0.1% SDS solution preheated to 65 °C were added thereto, placed in a hybridization chamber preheated to 65 °C, and shook gently for 20min × 2, followed by blenching for one time with 2 × SSC.

The hybridization membrane was removed to 1 × blocking solution (containing 1% blocking agent of maleic acid buffer: 0.1mol/L maleic acid, 0.15mol/L NaCl, pH7.5) and blocked at room temperature for 30min.

After removing the blocking solution, 1× blocking solution of 1:5000 dilution of anti-digoxigenin antibody was added thereto, incubating at room temperature for 30min. The membrane was washed twice for 15min each time with 200ml of maleic acid (containing 0.3% Tween-20 in 1 × maleic acid buffer) at room temperature. Then the membrane was washed in the test buffer (0.1M Tris-HCl, 0.1M NaCl, pH 9.5) for 5min. CSPD (25 mmol/L, 11.6 mg/ml) was diluted with the test buffer to 1:200. The fluid on the surface of the nylon membrane was absorbed completely with paper tissues, and the nylon membrane was placed on fresh keeping films. The diluted CSPD was added onto the nylon membrane in a volume of 1ml/100cm² and coated uniformly. The nylon membrane was wrapped tightly by fresh keeping film, and the reaction was performed at room temperature for 5min, followed by absorbing the excessive CSPD with filter paper and subsequently incubating at 37°C for 10min. The X-ray films and the nylon membrane wrapped by the fresh keeping films were layered in the dark box and exposed for 15-20min in the dark room. The X-ray films were rinsed, and the relative gray values of HBV replication intermediate in the transfected cells were determined using image analysis software Quantity One.

### 7. Determination of SEAP in the supernatant

Internal reference plasmid pSEAP2 was co-transfected and the activity of SEAP in the cell culture supernatant was determined to detect the transfection efficiency. Except for the experimental error, such as the difference of the number of the transfected plasmids and the transfected cells, which might cause the difference of the antigens expressed by viruses and the difference of replication and transcription of viruses, it might also identify whether the decrease of the indicators of viruses assays is caused by the decrease of the number of the transfected cells as a result of the ADV-induced cytotoxicity . To the same treated HepG2 cells co-transfected with SEAP plasmid, SEAP in the cell culture supernatant was detected. We found that the secretory values of SEAP were similar. The differential values between the secretory values were less than 5%-10%, suggesting that experimental error was negligible and the experimental results of each group could be compared directly. Detailed processes of detection were as follows:

100µl of cell culture supernatant was heated at 65 °C for 30min to inactivate endogenous alkaline phosphatase, and then centrifuged at 14,000×g for 2min to remove cell debris. (Supernatant can be stored at -20°C). 50µl of the treated cell culture supernatant was transferred to 96-well plates and balanced to 37°C. Multiple holes were set and 45µl of 2× SEAP buffer (20mmol/l homoarginine, 1mmol/L MgCl₂, 21% ethanolamine, pH 9.8) which had been balanced to 37°C and 5µl of substrate solution (120 mmol/L nitrophenol phosphate prepared by 2× SEAP buffer reagent) was added. The detection plate was placed at 37°C for 15 minutes to develop color, followed by determining absorbance values of A405.

### III. Experimental results

### 1. The detection results of the mutation sites of serum in clinically ADV-resistant patients

Among the 23 patients, 5 patients were detected for rtE218G mutation as well as rtA181V/T mutation and/or rtN236T mutation, and the rest 18 patients were only detected for rtE218G mutation.

### 2. Construction and identification of expression plasmids of rtE218G-mutated HBV

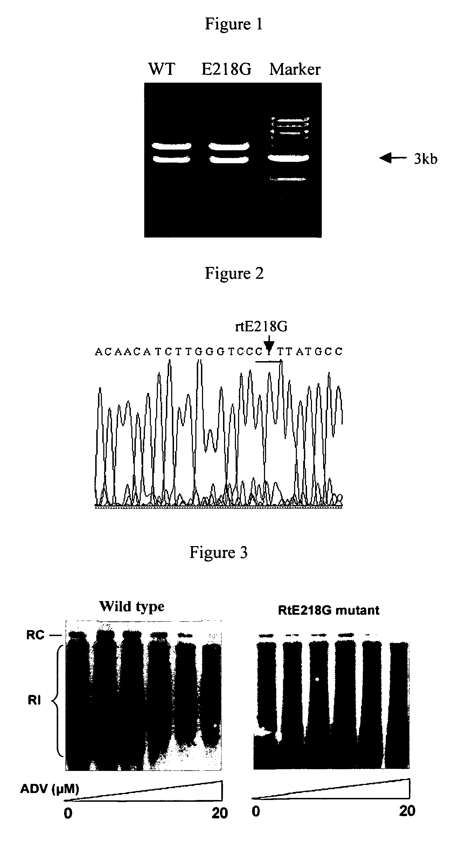

Wild-type HBV replication plasmid PUC-HBV1.2WT was taken as template and site-directed PCR technology was used to construct an expression plasmid of rtE218G mutant named as PUC-HBV1.2-E218G Identification of dual-enzyme digestion by *EcoR* I and *Hind* III was performed on mutagenic plasmids, and the results were shown in Figure 1; the mutation sites were identified by sequencing, and the results were shown in Figure 2. Compared with the wild-type, rtE218G mutation had been occurred in polymerase region, and the codon has been changed from GAG to GGG.

### 3. Expression of antigen in HepG2 cells which were transfected by expression plasmids of wild-type HBV and adefovir-resistant HBV mutation and the secretion level of virus particles in the supernatant.

HepG2 cells were transiently transfected by the wild-type and the mutagenic plasmids described above for 96 hours, and then the cells and the culture supernatant were collected. The transfection efficiency was corrected on the basis of the level of exogenous alkaline phosphatase expressed by plasmid pSEAP2, and high level of HbeAg, HBsAg was detected in the supernatants of the wild-type and the mutagenic HBV transfected cells group, and the number of viral DNA copies reached (4.52 ± 0.83) × 10⁵ (copies/ml) and (4.25 ± 0.14) × 10⁵ (copies/ml). This indicates that, after HepG2 cells were transfected by mutagenic plasmids, HBV replication status could be established, virus antigens were expressed and virus particles were secreted.

### 4. Comparation of replication capacity of the wild-type, rtE218G mutant strain

HepG2 cells were transiently transfected by the wild-type and the mutagenic plasmids for 96 hours, and then the levels of HBV DNA in the supernatant and HBV replication intermediates in the cell plasma were determined. The results showed that, the replication capacity of the mutant strain was less than the wild-type strain, which was 87% of that of the wild-type strain.

### 5. Sensitivity of wild-type, rtE218G mutants to adefovir

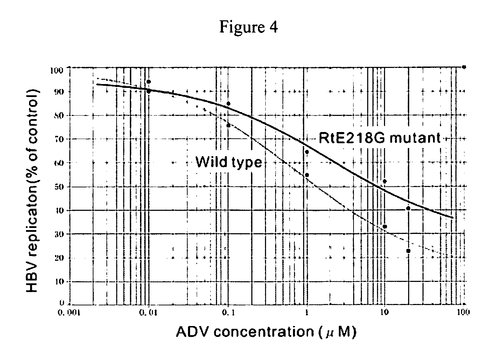

Adefovir with the final concentrations of 0, 0.01, 0.1, 1, 5, 10 and 20µM were respectively added to the HepG2 cells transiently transfected by the wild-type and the mutagenic plasmids described above, treating for 7 days. HBV replication intermediates in the cytoplasm were detected. The Southern blotting results were shown in Figure 3. It showed that, different concentrations of adefovir had the inhibitory effects on the replication intermediates of the wild-type strain and the mutant strain. After the analysis of the relative gray values, quantitative analysis was conducted on the basis of the effect of different concentrations of drugs on the level of replication intermediates. Each of the dose-response curves was depicted using SPSS10.0 statistical software, and the results were shown in Figure 4. The half maximal inhibitory concentration (IC50 values) of adefovir for the wild strain and the mutant strain were calculated, which were 1.7µM and 9.3µM respectively. The IC50 of rtE218G mutant strain was 5.5 times that of the wild strain.

### Example 2 Detection of mutation sites

In this case three different detection methods were provided to detect rtE218G mutation.

### 1. Polymerase chain reaction-restriction fragment length polymorphism (PCR-RFLP) analysis

According to the sequence of the reference strains of HBV genotypes in GenBank, mutagenic primer B2 (downstream of 5'-TTG GTA ATA GAG GTA AAA AGG TAC-3') was designed. The primer as well as rtE218G mutation formed the restriction site recognized by *Ban* I endonuclease. Additionally, upstream primer B1 (upstream 5'-GAG TGG GCC TCA GTC CGT TTC TC-3') was designed, and then the desired DNA fragments were amplified by PCR. The amplification conditions: 94 °C, 60s; 94°C, 30s; 60°C, 45s; 35 cycles; and the amplified DNA fragments were 160bp fragments. Ban I restriction endonuclease analysis was used to detect amplified DNA fragments. Digestion system: 10 × enzyme buffer, 1µl; enzyme, 5U; PCR products, 2-4µl; DEPC water was added to make a total volume of 10µl. Digestion conditions: 37°C, 2 ∼ 4 hours. As a result, the amplified DNA fragments of the mutant strain were digested into two fragments of 140bp and 20bp, and the amplified DNA fragments of the wild strain were not digested and remained the 160bp fragments.

### 2. Sequencing the nucleotide

The desired DNA fragments were amplified by nest PCR. Outer primers consist of upstream SA: 5'-TCG TGT TACAGGCGGGGT TT-3', downstream P0: 5'-GGG TTG CGT CAG CAA ACA CTT G-3'; inner primer consist of upstream BSE: 5'-CTC GTG GTG GAC TTC TCT CA-3', downstream 1162:5'-TTG CCG GGC AAC GGG GTA AAG-3'. The amplification conditions: 94°C, 300s; 94°C, 30s; 55°C, 30s; 72°C, 45s; 35 cycles. 3µl of 1:100 dilution of first PCR product was taken as template for the second PCR. 910bp of PCR product was obtained and sequenced directly; alternatively, A-T cloning could be carried out firstly, and then multiple colonies were picked and sequenced. Finally, it was directly determined whether the colony was a mutant strain by the sequencing results.

### 3. Method of sequence-specific primers:

According to the mutation sites, sequence-specific primers of F1: 5'-GAG TGG GCC TCA GTC CGT TTC TC-3', and F2: 5'-TTG GTA ATA GAG GTA AAA AGG TTT C-3' were designed and synthesized; the fluorescent probe: FAM-5'-TAG TGC CAT TTG TTC AGT GGT TCG TAG-TAMRA-3'. The desired DNA fragments were amplified by quantitative PCR. The amplification conditions: 42°C, 120s; 94°C, 120s; then 94°C, 10s; 65°C, 20s; 40 cycles. The results showed that, only mutant strain could be specifically amplified, since only the sequence of rtE218G mutation could combine complementally with the designed primers.

### Industrial applicability

The present invention discloses HBV rtE218G mutation sites which are found to be associated with adefovir resistance. The present invention further provides a method for detecting rtE218G mutation sites and the relevant reagents. By detecting the mutation sites, it is useful to guide the clinical drug administration and screen new drugs.

## Claims

1. HBV mutation strain, wherein amino acid codon at position 218 of the polymerase gene of the virus strain is mutated from GAG to GGG, i.e. rtE218G mutation occurs.

2. A method for identification of the HBV mutation strain according to claim 1, comprising the steps of :
1) amplifying the target DNA fragments containing rtE218G mutation site by PCR (polymerase chain reaction);
2) digesting the amplified products with restriction endonuclease, wherein the identification sequence of the restriction endonuclease contains the rtE218G mutation site;
3) subjecting the digestion products from step 2) to agarose gel electrophoresis, then determining whether rtE218G mutation occurs based on different digestion bands.

3. The method according to claim 2, wherein the primer pairs for amplifying the target DNA fragments in step 1) are B1 and B2 with the nucleotide sequences as shown in SEQ ID NO.1 and SEQ ID NO.2, respectively.

4. The method according to claim 2, wherein the restriction endonuclease in step 2) is *Ban* I endonuclease.

5. A method for identification of the HBV mutation strain according to claim 1, comprising the steps of :
1) amplifying the DNA fragments containing rtE218G mutation site by PCR;
2) sequencing the nucleotides of the amplified DNA fragments from step 1);
3) determining whether the rtE218G mutation occurs based on the sequencing results.

6. The method according to claim 5, wherein the PCR used in step 1) is nest-PCR.

7. The method according to claim 6, wherein the outer primer pairs used in the nest-PCR are SA and P0 with the nucleotide sequences as shown in SEQ ID NO.18 and SEQ ID NO.7, respectively.

8. The method according to claim 6, wherein the inner primers used in the nest-PCR are BSE and 1164 with the nucleotide sequences as shown in SEQ ID NO.6 and SEQ ID NO.19, respectively.

9. A method for identification of the HBV mutation strain according to claim 1, comprising the steps of:
1) designing and synthesizing the sequence-specific primers based on the mutation sites;
2) amplifying the target DNA fragments by PCR, and determining whether the rtE218G mutation occurs by detecting the existence of the amplified specific products.

10. The method according to claim 9, wherein the PCR used in step 2) is quantitative PCR.

11. The method according to claim 9, wherein the sequence-specific primer used in PCR amplification is F1 with the nucleotide sequence as shown in SEQ ID NO.3.

12. The method according to claim 11, wherein another primer used in pairs with F1 is F2 with the nucleotide sequence as shown in SEQ ID NO.4.

13. The method according to claim 10, wherein the fluorescent quantitative PCR uses the fluorescent labeled probes with the nucleotide sequence as shown in SEQ ID NO.5

14. A method for identification of the HBV mutation strain according to claim 1, wherein the probes directed to rtE218G mutation site are used to hybrid with the DNA fragments to be detected, determining whether the rtE218G mutation occurs by detecting the results of hybridization.

15. The method according to claim 14, wherein the probes are fixed on the blotting membrane, and the HBV DNA fragments having marker and containing the site to be detected are obtained by PCR method, followed by hybridizing the DNA fragments with the probes, washing the unhybridized DNA fragments, and determining whether rtE218G mutation occurs by detecting the results of hybridization.

16. The method according to claim 15, wherein the marker is a biotin marker, after the hybridization, streptavidin labeled with alkaline phosphatase is added to bind to the biotin labeled on the hybridization products, followed by adding BCIP/NBT for color development, and then determining whether rtE218G mutation occurs based on the hybridization results.

17. A method for identification of the HBV mutation strain according to claim 1, wherein two adjacent oligonucleotide chains complementing to the DNA of HBV mutation strain are designed for rtE218G, and the end of one of the oligonucleotide chains which adjacents to another chain is complementary to the bases of the rtE218G mutation site, the HBV DNA to be detected is taken as template to carry out ligase chain reaction, and the ligation products are detected to determine whether rtE218G mutation occurs.

18. A detection reagent, wherein the reagent contains primers and/or probes for detecting rtE218G mutation sites of HBV polymerase gene.

19. The detection reagent according to claim 18, which is selected from the group consisting of:
1) primer pairs, either (a) which specifically amplifies nucleotide sequences containing rtE218G mutation sites; or (b) whose amplified products contain rtE218G mutation sites which may constitute the identification sites of the restriction endonuclease with the sequence of one end or the sequences of both ends;
2) sequence-specific primer pairs, one of which specifically binds to the nucleotide sequence containing rtE218G mutation;
3) specific probes, which specifically bind to the nucleotide sequence containing rtE218G mutation;
4) oligonucleotide sequences for ligase chain reaction, wherein two pairs of adjacent oligonucleotide chains complementary to the DNA sequences of HBV mutation strain respectively are designed for rtE218G mutation, one oligonucleotide chain of each pair is adjacent to another, and the terminal bases of the adjacent ends are complementary to the bases of rtE218G mutation sites.

20. The detection reagent according to claim 19, wherein the primer pairs (b) in 1) are B1 and B2 with the nucleotide sequences as shown in SEQ ID NO.1 and SEQ ID NO.2, respectively.

21. The detection reagent according to claim 19, wherein sequence-specific primer pairs in 2) are F1 and F2 with the nucleotide sequences as shown in SEQ ID NO.3 and SEQ ID NO.4, respectively.

22. The detection reagent according to claim 19, wherein specific probes in 3) has the nucleotide sequence as shown in SEQ ID NO.5.

23. The detection reagent according to claim 19, wherein the detection reagent containing primer pairs (a) of 1) further contains the outer primers or the inner primers of primer pairs (a), composing the inner primers and outer primers of nest-PCR.

24. The detection reagent according to claim 19 or 23, wherein the primer pairs (a) are SA and P0 with the nucleotide sequences as shown in SEQ ID NO.18 and SEQ ID NO.7, respectively.

25. The detection reagent according to claim 24, wherein it further comprises inner primer pairs of BSE and 1164 with the nucleotide sequences as shown in SEQ ID NO.6 and SEQ ID NO.19, respectively.

26. A test kit comprising the detection reagents according to any one of claims 18 to 25.

27. The kit according to claim 26, wherein the kit further comprises the restriction endonuclease when it contains the primer pairs (b) in 1) according to claim 15; or the kit further comprises the fluorescent probes specific binding to the target sequences when it contains the sequence-specific primer pairs in 2) according to claim 15.

28. Use of HBV rtE218G mutations in guiding clinical administration.

29. Use of primer pairs B1 and B2 with the nucleotide sequences as shown in SEQ ID NO.1 and SEQ ID NO.2 in preparing a kit for guiding clinical administration by detecting HBV rtE218G mutations.

30. Use of primer pairs SA and P0 with the nucleotide sequences as shown in SEQ ID NO.18 and SEQ ID NO.7 in the preparing a kit for guiding clinical administration by detecting HBV rtE218G mutations.

31. Use of primers BSE and 1164 with the nucleotide sequences as shown in SEQ ID NO.6 and SEQ ID NO.19 in the preparing a kit for guiding clinical administration by detecting HBV rtE218G mutations.

32. Use of primers F1 and F2 with the nucleotide sequences as shown in SEQ ID NO.3 and SEQ ID NO.4 in the preparing a kit for guiding clinical administration by detecting HBV rtE218G mutations.

33. Use of fluorescent probes with the nucleotide sequence as shown in SEQ ID NO.5 in the preparing a kit for guiding clinical administration by detecting HBV rtE218G mutations.

34. A method for screening drugs, wherein it takes the mutation strain according to claim 1 as a target to conduct *in vitro* or *in vivo* screening for a drug inhibiting the replication of the mutation strain.

35. Carriers containing HBV mutation strain according to claim 1 or containing the HBV DNA of rtE218G

36. Host cells containing the carriers according to claim 35.

37. The host cells according to claim 36, wherein it is *Escherichia coli* PUC-HBV1.2-E218G strain with the deposit number of CGMCC No. 3079.
